# EUROPEAN PATENT APPLICATION

(11) **EP 4 019 631 A1**
(43) Date of publication of application: **29.06.2022**
(21) Application number: 20856117.5
(22) Date of filing: 19.08.2020
(51) Int. Cl.: C12N 15/09, C12Q 1/00, C12Q 1/6844, C12Q 1/6895, C12N 9/00, G01N 33/53, G01N 33/569

(54) **PRIMER PAIR FOR DETECTING PNEUMOCYSTIS JIROVECII, METHOD FOR DETECTING PNEUMOCYSTIS JIROVECII USING SAME AND REAGENT KIT THEREFOR**

(30) Priority: 23.08.2019 JP 2019152901
(71) Applicant: FUJIFILM Wako Pure Chemical Corporation, Osaka 540-8605 (JP)
(72) Inventor: TERASHIMA, Kazuhiro, Amagasaki-shi Hyogo 661-0963 (JP)
(74) Representative: Winter, Brandl - Partnerschaft mbB
(86) International application number: PCT/JP2020/031294
(87) International publication number: WO 2021/039540

(57) **Abstract**

An object of the present invention is to provide a primer pair for detecting *Pneumocystis jirovecii,* which has a low probability of false negatives and is excellent in sensitivity, a method for detecting *Pneumocystis jirovecii* using the same, and a reagent kit therefor.

The present invention relates to "a primer pair for detecting *Pneumocystis jirovecii,* which is selected from the group consisting of (i) a combination of a forward primer consisting of a base sequence represented by SEQ ID NO: 1 and a reverse primer consisting of a base sequence represented by SEQ ID NO: 2, (ii) a combination of the forward primer consisting of the base sequence represented by SEQ ID NO: 1 and a reverse primer consisting of a base sequence represented by SEQ ID NO: 4, and (iii) a combination of a forward primer consisting of a base sequence represented by SEQ ID NO: 3 and the reverse primer consisting of the base sequence represented by SEQ ID NO: 2".

SEQUENCE LISTING

<110>  FUJIFILM Wako Pure Chemical Corporation

<120>  A primer pair for detecting Pneumocystis jirovecii

<130>  2134PCT

<150>  JP2019-152901
<151>  2019-08-23

<160>  8

<170>  PatentIn version 3.5

<210>  1
<211>  32
<212>  DNA
<213>  Artificial Sequence

<220>
<223>  oligonucleotide primer

<400>  1
catatgattc tatattaatg gatgtggaga at                                    32


<210>  2
<211>  30
<212>  DNA
<213>  Artificial Sequence

<220>
<223>  oligonucleotide primer

<400>  2
tttatagcag gaataactcg agaaatctct                                       30


<210>  3
<211>  30
<212>  DNA
<213>  Artificial Sequence

<220>
<223>  oligonucleotide primer

<400>  3
catatgattc tatattaatg gatgtggaga                                       30


<210>  4
<211>  28
<212>  DNA
<213>  Artificial Sequence

<220>
<223>  oligonucleotide primer

<400>  4
tttatagcag gaataactcg agaaatct                                         28

<210>  5
<211>  142
<212>  DNA
<213>  Pneumocystis jirovecii

<400>  5
catatgattc tatattaatg gatgtggaga attttataaa tgcaggggcg acgataattg      60

atattggtgg gcagtctgca cggtctggtt cacatgttgt ttctatagag gaagagattt     120

ctcgagttat tcctgctata aa                                              142


<210>  6
<211>  358
<212>  DNA
<213>  Bacillus subtilis

<400>  6
cgcaatagag ggaagtggtt ccgacagaat aaaaatgcca tttgaagact ggctgtttat      60

taagctttat tgtaaacaaa caagagaaga agagctaatt gcttttgaaa tagcggattt     120

ttataaccag atttctgatc aatatccagt cagacatttc tttatgaggt atcgggatcc     180

aaagcctcat ataagactta gatttaatgg aaaagccgaa gtgctgtaca gcttgtttcc     240

ccaattattg aattggctga aaagcttaag agaaaaagga ctggtttcag agtctgttat     300

cactcaatac gagcgggaga tagaacgata tggcgggcta agccttatgg aggctgca       358


<210>  7
<211>  24
<212>  DNA
<213>  Artificial Sequence

<220>
<223>  oligonucleotide primer

<400>  7
aacgcaatag agggaagtgg ttcc                                             24


<210>  8
<211>  22
<212>  DNA
<213>  Artificial Sequence

<220>
<223>  oligonucleotide primer

<400>  8
tgcagcctcc ataaggctta gc                                               22

## Description

### Technical Field

The present invention relates to a primer pair for detecting *Pneumocystis jirovecii,* a method for detecting *Pneumocystis jirovecii* using the same, and a reagent kit therefor.

### Background Art

Pneumocystis pneumonia (PCP) is an opportunistic infection caused by the yeast-like fungus *Pneumocystis jirovecii.* In addition, PCP is known to develop in patients with weakened immunity due to use of steroids and biologicals, acquired immunodeficiency syndrome, or the like, and the mortality rate is said to be 30% to 35%.

Since *Pneumocystis jirovecii* cannot be cultured in an artificial medium, PCP tests have been performed mainly by observation with a microscope. In recent years, genetic tests have been introduced, and detection of *Pneumocystis jirovecii* in samples such as sputum and bronchial lavage fluid by carrying out a nucleic acid amplification reaction using a primer pair specific to *Pneumocystis jirovecii* has been performed (Non-Patent Literature 1).

### Citation List

### Non-Patent Literature

Non-Patent Literature 1: Clinical Microbiology and Infection, Volume 18, No.6, June 2012, 591-597

### Summary of Invention

### Technical Problem

However, since the detection method of Non-Patent Literature 1 has a low sensitivity, false negatives may occur.

An object of the present invention is to provide a primer pair for detecting *Pneumocystis jirovecii,* which has a low probability of false negatives and is excellent in sensitivity, a method for detecting *Pneumocystis jirovecii* using the same, and a reagent kit therefor.

### Solution to Problem

The present invention has been made for the purpose of solving the problems, and has the following configurations.
[1] A primer pair for detecting *Pneumocystis jirovecii,* which is selected from the group consisting of (i) a combination of a forward primer consisting of a base sequence represented by SEQ ID NO: 1 and a reverse primer consisting of a base sequence represented by SEQ ID NO: 2, (ii) a combination of the forward primer consisting of the base sequence represented by SEQ ID NO: 1 and a reverse primer consisting of a base sequence represented by SEQ ID NO: 4, and (iii) a combination of a forward primer consisting of a base sequence represented by SEQ ID NO: 3 and the reverse primer consisting of the base sequence represented by SEQ ID NO: 2.
[2] The primer pair according to [1], in which the primer pair consists of the combination of the forward primer consisting of the base sequence represented by SEQ ID NO: 1 and the reverse primer consisting of the base sequence represented by SEQ ID NO: 2.
[3] The primer pair according to [1] or [2], in which at least one of the forward primer or the reverse primer is labeled with a labeling substance.
[4] The primer pair according to [3], in which the labeling substance is selected from a fluorescent substance, a radioactive isotope, or an enzyme.
[5] A method for detecting *Pneumocystis jirovecii,* comprising: carrying out a nucleic acid amplification reaction using the primer pair according to any one of [1] to [4] and a nucleic acid in a sample as a template, and detecting an obtained nucleic acid amplification product.
[6] A reagent kit for detecting *Pneumocystis jirovecii,* comprising the primer pair according to any one of [1] to [4].

### Advantageous Effects of Invention

According to the primer pair for detecting *Pneumocystis jirovecii* of the present invention, the method for detecting *Pneumocystis jirovecii* using the same, and the reagent kit therefor, *Pneumocystis jirovecii* can be detected with high sensitivity and high accuracy. Therefore, false negatives caused by methods of the related art can be reduced.

### (Brief Description of Drawings)

Fig. 1 shows a detection result of *Pneumocystis jirovecii,* obtained by carrying out a nucleic acid amplification reaction (PCR) using a primer pair of the present invention obtained in Example 1.
Fig. 2 is a test result of the detection sensitivity of *Pneumocystis jirovecii* in the nucleic acid amplification reaction (PCR) using a primer pair of the present invention obtained in Example 2.
Fig. 3 is a test result of the detection sensitivity of *Pneumocystis jirovecii* in the nucleic acid amplification reaction (PCR) using a known primer pair obtained in Comparative Example 1.
Fig. 4 is a test result of the detection sensitivity of *Pneumocystis jirovecii* in the nucleic acid amplification reaction (PCR) using a primer pair of the present invention obtained in Example 3.
Fig. 5 is a test result of the detection sensitivity of *Pneumocystis jirovecii* in the nucleic acid amplification reaction (PCR) using the primer pair of the present invention obtained in Example 4.

### Description of Embodiments

### Primer pair for detecting Pneumocystis jirovecii of present invention

A primer pair for detecting *Pneumocystis jirovecii* of the present invention (hereinafter, also referred to as the primer pair of the present invention) is selected from the group consisting of (i) a combination of a forward primer consisting of a base sequence represented by SEQ ID NO: 1 and a reverse primer consisting of a base sequence represented by SEQ ID NO: 2, (ii) a combination of the forward primer consisting of the base sequence represented by SEQ ID NO: 1 and a reverse primer consisting of a base sequence represented by SEQ ID NO: 4, and (iii) a combination of a forward primer consisting of a base sequence represented by SEQ ID NO: 3 and the reverse primer consisting of the base sequence represented by SEQ ID NO: 2.

As the primer pair of the present invention, (i) a combination of the forward primer consisting of the base sequence represented by SEQ ID NO: 1 and the reverse primer consisting of the base sequence represented by SEQ ID NO: 2 is preferable.

The combinations (i) to (iii) will be described below.

Among the base sequences of *Pneumocystis jirovecii,* (i) the forward primer consisting of the base sequence represented by SEQ ID NO: 1 (GenbankID: XM_018374493, base numbers: 1478 to 1509) and the reverse primer consisting of the base sequence represented by SEQ ID NO: 2 (GenbankID: XM_018374493, base numbers: 1590 to 1619), (ii) the forward primer consisting of the base sequence represented by SEQ ID NO: 1 and the reverse primer consisting of the base sequence represented by SEQ ID NO: 4 (GenbankID: XM_018374493, base numbers: 1592 to 1619), and (iii) the forward primer consisting of the base sequence represented by SEQ ID NO: 3 (GenbankID: XM_018374493, base numbers: 1478 to 1507) and the reverse primer consisting of the base sequence represented by SEQ ID NO: 2 are respectively annealed to the base sequence represented by SEQ ID NO: 5 (GenbankID: XM_018374493, base numbers: 1478 to 1619).

Therefore, the base sequence represented by SEQ ID NO: 5 is amplified by carrying out the nucleic acid amplification reaction such as PCR using the primer pair (i), (ii) or (iii) and a nucleic acid-containing sample such as DNA derived from *Pneumocystis jirovecii.*

The design of the primer pair of the present invention may be carried out by a method known per se, which has been usually performed in the field, and specific examples thereof include a method of using software for primer design such as Primer3.

The primers constituting the primer pair of the present invention (hereinafter, also referred to as the primers according to the present invention) may be obtained by a method known per se, which has been usually performed in the field, and specific examples thereof include a method of preparing primers by a chemical synthesis method and a method of obtaining primers by a gene manipulation method using a vector and the like. Among these, from the viewpoint of easily obtaining a large amount of primers with a constant quality at a low cost, the method of preparing primers by a chemical synthesis method is preferable.

The primer according to the present invention may be labeled with a labeling substance. Specifically, at least one of the forward primer or the reverse primer in the primer according to the present invention may be labeled with a labeling substance.

The labeling substance used for labeling the primer according to the present invention is not limited as long as the labeling substance is a substance known per se, which has been usually used in the field, and specific examples thereof include a fluorescent substance, a radioactive isotope, and an enzyme. Among these, the fluorescent substance is preferable.

Examples of the fluorescent substance include TAMRA^{™} (manufactured by Sigma-Aldrich Co., LLC), Alexa555 and Alexa647 (both manufactured by Thermo Fisher Scientific Inc.), and Cyanine Dye-based Cy3 and Cy5 (manufactured by GE Healthcare), and Fluorescein. Among these, TAMRA^{™} is preferable.

Examples of the radioactive isotope include ³²P, ³³P, and ³⁵S.

Examples of the enzyme include alkaline phosphatase and horseradish peroxidase.

In the primer according to the present invention which has been labeled with the labeling substance, it should be noted that the labeling substance may be bound to the primer directly or via a linker. The linker is not limited as long as the linker is usually used in the field, and a nucleic acid of 1 to 3 bases may be used.

The primer according to the present invention may be labeled with the fluorescent substance by a method known per se, which has been usually performed in the field, and specific examples thereof include a method of incorporating a fluorescein-labeled nucleotide into the primer using a method known per se and a method of substituting a nucleotide having a linker arm in an oligonucleotide of a sequence (Nucleic Acids Res., 1986, Vol. 14, p.6115).

The primer according to the present invention may be labeled with a radioactive isotope by a method known per se, which has been usually performed in the field, and specific examples thereof include a method of labeling a primer by incorporating a nucleotide labeled with a radioactive isotope during synthesis of the primer and a method of labeling a primer with a radioactive isotope after synthesis of the primer. Specific examples thereof include a random primer method, nick translation, a 5' terminal labeling method using a T4 polynucleotide kinase, and a 3' terminal labeling method using a terminal deoxynucleotidyl transferase.

The primer according to the present invention may be labeled with an enzyme by a method known per se, which has been usually performed in the field, and specific examples thereof include a direct labeling method of covalently binding an enzyme molecule such as alkaline phosphatase or horseradish peroxidase directly to the primer to be labeled.

In addition, the labeling substance may be bound to the primer of the present invention according to a detection system using a biotin-avidin reaction. In this case, the binding may be performed by the method described in E. P. Diamandis, T. K. Christopoulos, Clinical Chemistry 1991, 37, pp. 625 to 636.

### Method for detecting Pneumocystis jirovecii of present invention

A method for detecting *Pneumocystis jirovecii* of the present invention (hereinafter, also referred to as the detection method of the present invention) includes carrying out a nucleic acid amplification reaction using the primer pair of the present invention and a nucleic acid in a sample as a template, and detecting the obtained nucleic acid amplification product.

Specific examples of the sample in the detection method of the present invention include saliva, sputum, induced sputum, suction sputum, bronchial lavage fluid, bronchoalveolar lavage fluid, nasopharyngeal aspirate, oral lavage fluid, and nasal cavity wiping sample. Operations such as concentration and separation of *Pneumocystis jirovecii* present in the sample, extraction and purification of a nucleic acid from *Pneumocystis jirovecii,* and the like may be performed on these samples before use for the detection method of the present invention.

The concentration and the separation of *Pneumocystis jirovecii* present in the sample may be performed by a method known per se, which has been usually performed in the field, and specific examples thereof include filtration and centrifugation.

The extraction and the purification of the nucleic acid from the *Pneumocystis jirovecii* may be performed by a method known per se, which has been usually performed in the field, and specific examples include a method of using phenol and chloroform after destroying the cell wall of *Pneumocystis jirovecii* and a method of using alcohol such as ethanol or isopropanol after destroying the cell wall of *Pneumocystis jirovecii.*

It should be noted that the cell wall of *Pneumocystis jirovecii* may be destroyed by a method known per se, which has been usually performed in the field, and specific examples thereof include a method of using a surfactant such as SDS or a protein denaturing agent such as guanidine thiocyanate and a method of physically crushing the cell wall with glass beads or the like.

The nucleic acid in the sample in the detection method of the present invention is a nucleic acid derived from *Pneumocystis jirovecii* present in the sample, and examples thereof include DNA and RNA. Among these, DNA is preferable.

It should be noted that in a case where the nucleic acid is RNA, complementary DNA (cDNA) may be synthesized by a reverse transcription reaction such as a transcription-reverse transcription concerted reaction (TRC) method and subjected to a nucleic acid amplification reaction described below.

The primer pair used in the detection method of the present invention is as described in the section of <Primer pair for detecting *Pneumocystis jirovecii* of present invention>, and specific examples, preferred examples, and the like are also the same as described above.

Further, in the detection method of the present invention, the nucleic acid amplification reaction described below may be carried out using a primer pair for detecting an internal control in addition to the primer pair of the present invention.

The internal control may be any bacterium other than *Pneumocystis jirovecii,* and examples thereof include bacteria such as Bacillus subtilis, Bacillus cereus, and Clostridium difficile. Among these, the Bacillus subtilis is preferable.

In addition, each of the primers constituting the primer pair for detecting the bacterium selected as the internal control may be labeled with a labeling substance. Specifically, at least one of the forward primer or the reverse primer in the primer pair may be labeled with a labeling substance.

It should be noted that examples of the labeling substance and the method for labeling the primer with the labeling substance are the same as described in the section of <Primer pair for detecting *Pneumocystis jirovecii* of present invention>, and specific examples, preferred examples, and the like are also the same as described above.

The nucleic acid amplification reaction in the detection method of the present invention may be carried out by a method known per se, which has been usually performed in the field, and specific examples thereof include a polymerase chain reaction (PCR) method, a transcription -mediated amplification (TMA) method, a strand displacement amplification (SDA) method, and a loop-mediated isothermal amplification (LAMP) method. Among these, the PCR method is preferable.

A reagent used for the nucleic acid amplification reaction in the detection method of the present invention may be any reagent known per se, which has been usually used in the field, and specific examples thereof include a nucleic acid synthase such as DNA polymerase [such as KOD Exo (-) (manufactured by Toyobo Co., Ltd.) or KOD Hot Start (manufactured by Toyobo Co., Ltd.)], a nucleic acid synthetic substrate such as dNTP, a buffer solution such as Tris-HCl or K₂HPO₄, and a salt such as MgCl₂, KCl, or (NH₄)₂SO₄.

In addition, arbitrary components, for example, a surfactant such as polyethylene glycol, Triton (manufactured by The Dow Chemical Company), Nonidet (manufactured by Shell Chemical Japan Ltd.), or CHAPS (manufactured by Dojin Chemical Laboratory), a preservative such as PROCLIN 300, and a polypeptide such as bovine serum albumin (BSA) may be contained in addition to the reagent unless the nucleic acid amplification reaction in the detection method of the present invention is not inhibited.

The detection of the nucleic acid amplification product in the detection method of the present invention may be carried out by a method known per se, which has been usually performed in the field, and specific examples thereof include an end point method and a real-time method. Among these, the real-time method is preferable.

The end point method is a method for separating and detecting an amplification product obtained by the nucleic acid amplification reaction using the primer pair of the present invention.

Meanwhile, the real-time method is a method for detecting an amplification product obtained by the nucleic acid amplification reaction using the primer pair of the present invention in a real time during the nucleic acid amplification reaction.

Specific methods of the end point method and the real-time method include a labeled primer method (a), an intercalator method (b), and a labeled probe method (c). Among these, the labeled primer method (a) is preferable.

Hereinafter, the methods (a), (b), and (c) will be described.

### Labeled primer method (a)

The labeled primer method (a) is, for example, performed in the following manner.

"The nucleic acid amplification reaction is carried out using the nucleic acid in the sample as a template with the primer pair of the present invention, in which at least one of the primer pairs of the present invention is labeled with a labeling substance. Next, (i) the obtained amplification product is separated after the nucleic acid amplification reaction, and the label in the amplification product is detected (end point method), or (ii) the label in the amplification product obtained every time the nucleic acid amplification reaction is carried out for one to three cycles is detected in a real time (real-time method). As a result, in a case where the fluorescence derived from the label of the amplification product is detected, it is determined that the sample is positive for *Pneumocystis jirovecii*".

Between the methods (i) and (ii) in the labeled primer method, the method (ii) is preferable to the method (i).

In addition, as the method of "detecting the label in real time" in the labeled primer method, the fluorescence derived from the label of the amplification product may be detected after the amplification product obtained every time the nucleic acid amplification reaction is carried out for one to three cycles is once separated. In addition, the separation is as described below.

It should be noted that "detecting the label" in the present specification denotes that the labeling substance is directly or indirectly measured based on the properties of the labeling substance.

Specific examples of the method for the separation in the labeled primer method include methods known per se, such as electrophoresis and high performance liquid chromatography (HPLC). Among such methods, electrophoresis is preferable.

Specific examples of the electrophoresis include capillary electrophoresis, agarose gel electrophoresis, polyacrylamide gel electrophoresis (slab electrophoresis), starch gel electrophoresis, and isoelectric focusing electrophoresis. Among these, capillary electrophoresis is preferable.

It should be noted that the capillary electrophoresis may be performed by, for example, the methods known per se, described in WO2007/027495, WO2011/118496, WO2008/075520, and the like.

### Intercalator method (b)

The intercalator method (b) is, for example, carried out in the following manner in a case of being performed by the end point method.

"The nucleic acid amplification reaction is carried out using the nucleic acid in the sample as a template with the primer pair of the present invention. The obtained amplification product is separated. Next, the amplification product is stained with an intercalator, and the fluorescence derived from the intercalator is detected. As a result, in a case where the fluorescence derived from the intercalator is detected, it is determined that the sample is positive for *Pneumocystis jirovecii*".

In addition, as another aspect, the intercalator method (b) is carried out in the following manner in a case of being performed by the real-time method.

"The nucleic acid amplification reaction is carried out using the nucleic acid in the sample as a template with the primer pair of the present invention and the intercalator. Next, the fluorescence derived from the intercalator that intercalates in correlation with the amplification amount of the obtained amplification product is detected. As a result, in a case where the fluorescence derived from the intercalator is detected, it is determined that the sample is positive for *Pneumocystis jirovecii*".

The separation in the intercalator method is as described in the section of the labeled primer method (a), and the specific examples, the preferred examples, and the like are the same as described above.

The intercalator in the intercalator method may be any intercalator as long as the intercalator is known per se, which has been usually used in the field, and specific examples thereof include those described in WO2017/170376. Among the examples, SYTOX (trademark)-based dyes [for example, SYBR Gold (trademark), SYBR Green I (trademark), SYBR Green II (trademark), SYTOX Green (trademark), SYTOX Blue (trademark), and SYTOX Orange (trademark) (all manufactured by Thermo Fisher Scientific Inc.) are preferable, and SYBR Green I is more preferable.

### Labeled probe method (c)

The labeled probe method (c) is, for example, carried out in the following manner in a case of being performed by the end point method.

"The nucleic acid amplification reaction is carried out using the nucleic acid in the sample as a template with the primer pair of the present invention. The obtained amplification product is separated. Next, the amplification product is treated with a basic solution such as sodium hydroxide to form a single strand. Next, the amplification product is hybridized with a probe labeled with a labeling substance having a base sequence complementary to the base sequence of all or part of the amplification product to form a hybrid material, and the label in the hybrid material is detected. As a result, in a case where the label in the hybrid material is detected, it is determined that the sample is positive for *Pneumocystis jirovecii".*

The labeling substance and the method of performing labeling with the labeling substance in the labeled probe method are as described in the section of <Primer pair for detecting *Pneumocystis jirovecii* of present invention>, and specific examples, preferred examples, and the like are also the same as described above.

In addition, as another aspect, the labeled probe method (c) is carried out in the following manner in a case of being performed by the real-time method.

"The nucleic acid amplification reaction is carried out using the nucleic acid in the sample as a template with the primer pair of the present invention and the fluorescent labeled probe, and in a case where the fluorescence derived from the probe is detected, it is determined that the sample is positive for *Pneumocystis jirovecii".*

It should be noted that the fluorescent labeled probe denotes a probe which is designed to hybridize to a region amplified by the nucleic acid amplification reaction using the primer pair of the present invention and in which the 5' terminal thereof is labeled with, for example, a fluorescent dye (reporter fluorescent dye) and the 3' terminal thereof is labeled with, for example, a quencher dye.

The separation in the labeled probe method is as described in the section of the labeled primer method (a), and the specific examples, the preferred examples, and the like are the same as described above.

Preferred specific examples of the detection method of the present invention will be described.

First, DNA is extracted from a sample for detecting *Pneumocystis jirovecii* by a method known per se.

Meanwhile, for example, the primer pair of the present invention (for example, a forward primer consisting of the base sequence represented by SEQ ID NO: 1 and a reverse primer consisting of the base sequence represented by SEQ ID NO: 2) is synthesized by the phosphoramidite method using a DNA synthesizer. Next, at least one of the primer pair of the present invention is labeled with a labeling substance (for example, a fluorescent substance) by a method known per se.

The nucleic acid amplification reaction is carried out on DNA extracted from the sample in the following manner (for example, the PCR method) using the primer pair of the present invention which has been labeled with the labeling substance.

That is, 1 to 10 mM of a buffer solution (for example, a Tris-HCl buffer solution) having a pH of 7 to 9 which contains 0.1 µM to 2 µM of each primer constituting the primer pair of the present invention, 1 mM to 4 mM of a salt (for example, MgCl₂), 0.05 mg/mL to 10 mg/mL of a polypeptide (for example, BSA), 0.1% to 5% of a surfactant (for example, CHAPS), 0.1% to 5% of a preservative (for example, PROCLIN 300), 0.1 mM to 1 mM of a nucleic acid synthetic substrate (for example, dATP, dCTP, dGT, or dTTP), and 0.01 U/µL to 0.1 U/µL of a nucleic acid synthase (for example, a DNA polymerase) is prepared and used as a nucleic acid amplification reaction solution. Next, 0.1 ng to 100 ng of the DNA is added to 5 µL to 100 µL of the reaction solution.

The nucleic acid amplification reaction (for example, the PCR method) is carried out using the reaction solution and a nucleic acid amplification device such as a thermal cycler.

That is, after the reaction solution is heated at 93°C to 98°C for 25 seconds to 3 minutes, a cycle of heating at (1) 93°C to 98°C for 1 second to 30 seconds → (2) 50°C to 70°C for 5 seconds to 30 seconds → (3) 60°C to 80°C for 3 to 30 seconds is set as one cycle, and 30 to 50 cycles of heating is performed.

Next, the label in the amplification product obtained every time the nucleic acid amplification reaction is carried out for one to three cycles is detected in a real time.

As a result, in a case where the fluorescence derived from the label in the obtained amplification product is detected, it is determined that "the sample is positive for *Pneumocystis jirovecii".*

### Reagent kit for detecting Pneumocystis jirovecii of present invention

A reagent kit for detecting *Pneumocystis jirovecii* of the present invention (hereinafter, also referred to as the kit of the present invention) contains the primer pair of the present invention.

The primer pair contained in the kit of the present invention is as described in the section of <Primer pair for detecting *Pneumocystis jirovecii* of present invention>, and the specific examples, the preferred examples, and the like are also the same as described above.

The kit of the present invention may contain a primer pair for detecting an internal control.

The reagent kit of the present invention may contain reagents which have been usually used in the field, for example, a buffer solution (such as a tris buffer solution, a phosphoric acid buffer solution, a veronal buffer solution, a boric acid buffer solution, or a good buffer solution), a stabilizer, a preservative, and the like that do not inhibit the stability of coexisting reagents and the like and do not inhibit the nucleic acid amplification reaction such as PCR and the hybridization reaction. In addition, the concentration may be appropriately selected from the concentration range usually used in this field.

In addition, the reagent kit of the present invention may contain a reagent used for the nucleic acid amplification reaction such as a nucleic acid synthase, a nucleic acid synthetic substrate, an intercalator, a labeling substance, or a probe, an electrophoresis tank, a gel, and a reagent for electrophoresis such as a DNA marker, as necessary.

It should be noted that the nucleic acid synthase, the nucleic acid synthetic substrate, the intercalator, the labeling substance, and the probe are as described in the section of <Method for detecting *Pneumocystis jirovecii* of present invention>, and the specific examples, the preferred examples, and the like are also the same as described above.

Further, the reagent kit of the present invention may contain a manual for determining the presence or absence of the detection method of the present invention and *Pneumocystis jirovecii.* The manual denotes the instruction manual, the attached text, the pamphlet, and the like of the kit of the present invention in which the features, the principles, the operation procedures, the determination procedures, and the like of the detection method of the present invention are substantially described by means of texts, charts, and the like.

The reagent kit of the present invention may be accommodated in a container, and examples thereof include a container accommodating the primer pair of the present invention.

In addition, as described above, the reagent kit of the present invention, the primer pair of the present invention, and the reagents may be accommodated in an identical container, or a plurality of containers are prepared and the reagent kit, the primer pair, and the reagents may be accommodated in the containers separately or in combination.

### Method for determining Pneumocystis pneumonia according to present invention

A method for determining Pneumocystis pneumonia according to the present invention (hereinafter, also referred to as the determination method according to the present invention) is performed by carrying out the nucleic acid amplification reaction using the nucleic acid in the sample as a template with the primer pair of the present invention, detecting the obtained nucleic acid amplification product, and determining Pneumocystis pneumonia based on the detection result.

The primer pair in the determination method according to the present invention is as described in the section of <Primer pair for detecting *Pneumocystis jirovecii* of present invention>, and the specific examples, the preferred examples, and the like are also the same as described above.

The sample, the nucleic acid amplification reaction, the detection of the nucleic acid amplification product, and the like in the determination method according to the present invention are as described in the section of "<Method for detecting *Pneumocystis jirovecii* of present invention>, and the specific examples, the preferred examples, and the like are also the same as described above.

The determination of Pneumocystis pneumonia in the determination method according to the present invention is performed, for example, in the following manner based on the detection result of the nucleic acid amplification product obtained by the nucleic acid amplification reaction using the primer pair of the present invention.

That is, (i) in a case where a signal (fluorescence or the like) derived from the product amplified by the primer pair of the present invention is detected, it is possible to determined that "the test animal derived from the sample may be suffering from Pneumocystis pneumonia or the test animal is highly likely to be suffering from Pneumocystis pneumonia", and (ii) in a case where no signal (fluorescence or the like) derived from the product amplified by the primer pair of the present invention is detected, it is possible to determined that "the test animal derived from the sample is not suffering from Pneumocystis pneumonia or the test animal is unlikely to be suffering from pneumocystis pneumonia".

### Method for obtaining data for determining Pneumocystis pneumonia according to present invention

The method for obtaining data for determining pneumocystis pneumonia according to the present invention (hereinafter, also referred to as the method for obtaining data according to the present invention) is performed by carrying out the nucleic acid amplification reaction using the nucleic acid in the sample as a template with the primer pair of the present invention and detecting the obtained nucleic acid amplification product.

The primer pair in the method for obtaining data according to the present invention is as described in the section of <Primer pair for detecting *Pneumocystis jirovecii* of present invention>, and the specific examples, the preferred examples, and the like are also the same as described above.

The sample, the nucleic acid amplification reaction, the detection of the nucleic acid amplification product, and the like in the method for obtaining data according to the present invention are as described in the section of <Method for detecting *Pneumocystis jirovecii* of present invention>, and the specific examples, the preferred examples, and the like are also the same as described above.

Examples of the data in the method for obtaining data according to the present invention include (i) the detection result of the nucleic acid amplification product obtained by the method for obtaining data according to the present invention and (ii) the data suggesting that the test animal is likely to be suffering from Pneumocystis pneumonia or the test animal is highly likely to be suffering from Pneumocystis pneumonia.

### Method for supporting determination of Pneumocystis pneumonia according to present invention

The method for supporting the determination of Pneumocystis pneumonia according to the present invention (hereinafter, also referred to as the support method according to the present invention) is performed by carrying out the nucleic acid amplification reaction using the nucleic acid in the sample as a template with the primer pair of the present invention, detecting the obtained nucleic acid amplification product, and supporting the determination of Pneumocystis pneumonia based on the detection result.

The primer pair in the support method according to the present invention is as described in the section of <Primer pair for detecting *Pneumocystis jirovecii* of present invention>, and the specific examples, preferred examples, and the like are also the same as described above.

The sample, the nucleic acid amplification reaction, the detection of the nucleic acid amplification product, and the like in the support method according to the present invention are as described in the section of <Method for detecting *Pneumocystis jirovecii* of present invention>, and the specific examples, the preferred examples, and the like are also the same as described above.

The determination of Pneumocystis pneumonia in the support method according to the present invention is as described in the section of <Method for determining Pneumocystis pneumonia according to present invention>, and the specific examples, the preferred examples, and the like are also the same as described above.

The support method according to the present invention can be used as a method for supporting a medical worker such as a doctor in determining or diagnosing Pneumocystis pneumonia.

### Method for determining and treating Pneumocystis pneumonia according to present invention

The method for determining and treating Pneumocystis pneumonia according to the present invention (hereinafter, also referred to as the treatment method according to the present invention) is performed by carrying out the nucleic acid amplification reaction using the nucleic acid in the sample as a template with the primer pair of the present invention, detecting the obtained nucleic acid amplification product, determining Pneumocystis pneumonia based on the detection result, and appropriately treating the patient determined to have a risk of Pneumocystis pneumonia or a high risk of Pneumocystis pneumonia based on the determination result.

The primer pair in the treatment method according to the present invention is as described in the section of <Primer pair for detecting *Pneumocystis jirovecii* of present invention>, and the specific examples, the preferred examples, and the like are also the same as described above.

The sample, the nucleic acid amplification reaction, the detection of the nucleic acid amplification product, and the like in the treatment method according to the present invention are as described in the section of <Method for detecting *Pneumocystis jirovecii* of present invention>, and the specific examples, the preferred examples, and the like are also the same as described above.

The determination of Pneumocystis pneumonia in the treatment method according to the present invention is as described in the section of <Method for determining Pneumocystis pneumonia according to present invention>, and the specific examples, the preferred examples, and the like are also the same as described above.

Specific examples of the appropriate treatment in the treatment method according to the present invention include drug therapy performed by administering a drug such as a sulfamethoxazole/trimethoprim (ST) mixture.

### Device for determining Pneumocystis pneumonia according to present invention

A device for determining Pneumocystis pneumonia according to the present invention (hereinafter, also referred to as the device according to the present invention) includes at least a nucleic acid amplification reaction unit (1) and a detection unit (2). Further, the device may include a nucleic acid extraction unit (3), a determination unit (4), an output unit (5), and an input unit (6).

The nucleic acid amplification reaction unit (1) in the device according to the present invention is configured to perform the nucleic acid amplification reaction using the primer pair of the present invention.

The detection unit (2) in the device according to the present invention is configured to detect the nucleic acid amplification product obtained in the nucleic acid amplification reaction unit (1).

It should be noted that the nucleic acid amplification reaction unit (1) and the detection unit (2) may be configured independently or integrally, and specific examples thereof include the microfluidic device described in JP2018-89611A.

The nucleic acid extraction unit (3) in the device according to the present invention is configured to extract and/or purify the nucleic acid from the sample. Specifically, for example, the nucleic acid extraction unit (3) is configured to perform the method for extracting a nucleic acid described in WO2016/079981A and the method for purifying a nucleic acid described in WO2015/157650A.

The determination unit (4) in the device according to the present invention is configured to determine Pneumocystis pneumonia based on the result obtained by the detection unit (2).

The output unit (5) in the device according to the present invention is configured to output the result obtained by the nucleic acid amplification reaction unit (1), the detection unit (2), the nucleic acid extraction unit (3), and/or the determination unit (4).

The input unit (6) in the device according to the present invention is configured to send a signal for operating the nucleic acid amplification reaction unit (1) and/or the nucleic acid extraction unit (3) to the nucleic acid amplification reaction unit (1) and/or the nucleic acid extraction unit (3).

The primer pair in the device according to the present invention is as described in the section of <Primer pair for detecting *Pneumocystis jirovecii* of present invention>, and the specific examples, the preferred examples, and the like are also the same as described above.

The sample, the nucleic acid amplification reaction, the detection of the nucleic acid amplification product, and the like in the device according to the present invention are as described in the section of <Method for detecting *Pneumocystis jirovecii* of present invention>, and the specific examples, the preferred examples, and the like are also the same as described above.

The determination of Pneumocystis pneumonia in the device according to the present invention is as described in the section of <Method for determining Pneumocystis pneumonia according to present invention>, and the specific examples, the preferred examples, and the like are also the same as described above.

Hereinafter, the present invention will be described in detail based on the following examples, but the present invention is not limited to such examples.

### Examples

### Example 1. Detection of Pneumocystis jirovecii using primer pair of present invention

The nucleic acid amplification reaction (PCR) was carried out using the primer pair of the present invention to detect *Pneumocystis jirovecii.*

### (1) Determination of target sequence

Target sequence A:
   Among the base sequences of *Pneumocystis jirovecii,* the base sequence represented by SEQ ID NO: 5 (142 base pairs) was used as the target sequence.
Target sequence B:
   Bacillus subtilis was selected as the internal control, and the base sequence represented by SEQ ID NO: 6 (358 base pairs, GenbankID: CP034943.1) among the base sequences of Bacillus subtilis was used as the target sequence.

### (2) Design and synthesis of primer

From the target sequence A determined in the determination (1), a forward primer consisting of the base sequence represented by SEQ ID NO: 1 and a reverse primer consisting of the base sequence represented by SEQ ID NO: 2 were designed as a primer for the nucleic acid amplification reaction (PCR).

In addition, from the target sequence B determined in the determination (1), a forward primer consisting of the base sequence represented by SEQ ID NO: 7 and a reverse primer consisting of the base sequence represented by SEQ ID NO: 8 were designed as a primer for the nucleic acid amplification reaction (PCR).

The synthesis of the primers was outsourced to Fasmac Co., Ltd., thereby obtaining each of the primers. Next, the 5' terminal of the obtained reverse primer consisting of the base sequence represented by SEQ ID NO: 2 was labeled with a fluorescent substance (TAMRA^{™}). In addition, the 5' terminal of the forward primer consisting of the base sequence represented by SEQ ID NO: 7 was labeled with a fluorescent substance (TAMRA^{™}).

### (3) Sample preparation, nucleic acid amplification reaction (PCR), and electrophoresis

A DNA-containing sample was prepared from bronchial lavage fluid derived from a clinical specimen positive for *Pneumocystis jirovecii* using a fully-automated genetic analyzer µTASWako g1 (manufactured by FUJIFILM Wako Pure Chemical Corporation) according to the instruction manual of the device. Next, the nucleic acid amplification reaction (PCR) and electrophoresis were performed according to the instruction manual of the device. It should be noted that reaction solutions for PCR and the reaction conditions are as follows.

### • Reaction solution for PCR

Reaction solutions for PCR, containing the reagents listed in Table 1 were prepared.

**Table 1**

| **Reagent name** | **Concentration** |
|---|---|
| Forward primer (SEQ ID NO: 1) | 0.6 µM |
| Reverse primer (SEQ ID NO: 2) | 0.6 µM |
| Forward primer (SEQ ID NO: 7) | 0.6 µM |
| Reverse primer (SEQ ID NO: 8) | 0.6 µM |
| KOD Buffer (manufactured by Toyobo Co., Ltd.) | 1× |
| dNTP Mixture (manufactured by Toyobo Co., Ltd.) | 0.2 mM |
| KOD Hot Start (manufactured by Toyobo Co., Ltd.) | 0.06 U/µL |
| CHAPS (manufactured by Dojin Chemical Laboratory) | 0.50% |
| PEG6000 (manufactured by FUJIFILM Wako Pure Chemical Corporation) | 0.50% |
| BSA (manufactured by Sigma-Aldrich Co., LLC) | 0.1 mg/mL |
| PROCLIN 300 (manufactured by Sigma-Aldrich Co., LLC) | 0.20% |

### • Conditions for PCR reaction

After the reaction solution was heated at 98°C for 30 seconds, a cycle of heating at (1) 98°C for 3 seconds → (2) 62°C for 8 seconds → (3) 72°C for 6 seconds was set as one cycle, and 44 cycles of heating was performed.

### (5) Results

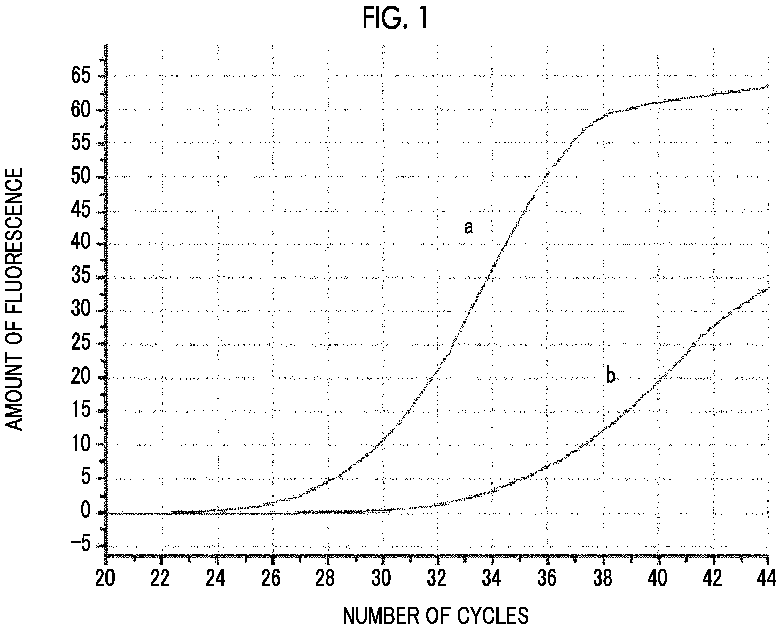

The obtained results are shown in Fig. 1. It should be noted that a in Fig. 1 denotes an amplification curve derived from *Pneumocystis jirovecii,* and b in Fig. 1 denotes an amplification curve derived from Bacillus subtilis. In addition, the vertical axis in Fig. 1 denotes the fluorescence intensity, and the horizontal axis in Fig. 1 denotes the number of PCR cycles.

As is apparent in Fig. 1, a fluorescent signal indicating the presence of the amplification product was able to be confirmed as a result of performing the nucleic acid amplification reaction (PCR) using the primer pair of the present invention. That is, it was possible to determine that the sample used this time was positive for *Pneumocystis jirovecii.*

In addition, a fluorescent signal indicating the presence of the amplification product was able to be confirmed as a result of performing the nucleic acid amplification reaction (PCR) using a primer pair specific to the internal control (Bacillus subtilis), and thus it was confirmed that the experimental system was performed appropriately.

As described above, it was found that *Pneumocystis jirovecii* was able to be detected by performing the nucleic acid amplification reaction (PCR) using the primer of the present invention. Further, it was found that *Pneumocystis jirovecii* was able to be detected by using the primer pair of the present invention without being affected by the presence of the primer pair specific to the internal control (Bacillus subtilis).

### Example 2. Test -1 of detection sensitivity in detection of Pneumocystis jirovecii using primer pair of present invention

The detection sensitivity of *Pneumocystis jirovecii* in the nucleic acid amplification reaction (PCR) using the primer pair of the present invention was tested.

### (1) Determination of target sequence

Similar to Example 1, the base sequence (142 base pairs) represented by SEQ ID NO: 5 among the base sequences of *Pneumocystis jirovecii* was used as the target sequence.

### (2) Design and synthesis of primer

A forward primer consisting of the base sequence represented by SEQ ID NO: 1 and a reverse primer consisting of the base sequence represented by SEQ ID NO: 2 were designed and synthesized in the same manner as in Example 1 (except that none of the primers used in Example 2 were labeled with the fluorescent substance).

### (3) Preparation of sample

With artificially synthesized DNA having the same base sequence as the target sequence, the DNA was diluted to 10 copies/µL, 10² copies/µL, 10³ copies/µL, 10⁴ copies/µL, and 10⁵ copies/µL, thereby preparing each of the samples.

### (4) Nucleic acid amplification reaction (PCR)

Reaction solutions for PCR, containing the reagents listed in Table 2 below were prepared, and 25 µL of the samples prepared in the preparation (3) of Example 2 were added, thereby obtaining samples for PCR.

**Table 2**

| **Reagent name** | **Concentration** |
|---|---|
| Forward primer (SEQ ID NO: 1) | 0.4 µM |
| Reverse primer (SEQ ID NO: 2) | 0.4 µM |
| KOD Buffer (manufactured by Toyobo Co., Ltd.) | 1× |
| dNTP Mixture (manufactured by Toyobo Co., Ltd.) | 0.3 mM |
| KOD Hot Start (manufactured by Toyobo Co., Ltd.) | 0.06 U/µL |
| CHAPS (manufactured by Dojin Chemical Laboratory) | 0.50% |
| PEG6000 (manufactured by FUJIFILM Wako Pure Chemical Corporation) | 0.50% |
| BSA (manufactured by Sigma-Aldrich Co., LLC) | 0.1 mg/mL |
| PROCLIN 300 (manufactured by Sigma-Aldrich Co., LLC) | 0.20% |
| SYBR Green I (manufactured by LONZA Bioscience) | 1× |

Next, each of the samples for PCR was dispensed into a 96-well plate, and PCR was performed using StepOnePlus^{™} (manufactured by Applied Biosystems). After the sample was heated at 97°C for 2 minutes, the reaction was carried out such that a cycle of heating at (1) 97°C for 10 seconds → (2) 63°C for 10 seconds → (3) 72°C for 10 seconds was set as one cycle and 40 cycles of heating was performed, and the amount of fluorescence derived from the amplification product labeled in each cycle was measured.

### (5) Results

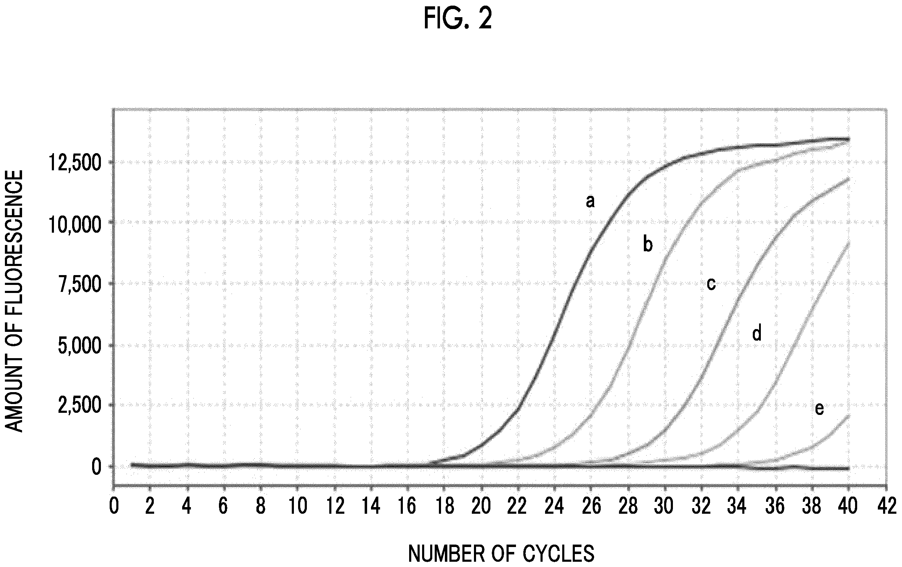

The obtained results are shown in Fig. 2. It should be noted that a to e in Fig. 2 each denote an amplification curve in a case of using samples diluted to 10⁵ copies/µL, 10⁴ copies/µL, 10³ copies/µL, 10² copies/µL, and 10 copies/µL. In addition, the vertical axis in Fig. 2 denotes the fluorescence intensity, and the horizontal axis in Fig. 2 denotes the number of PCR cycles.

As is apparent in Fig. 2, a fluorescent signal indicating the presence of the amplification product was able to be confirmed in all cases of a to e according to the nucleic acid amplification reaction (PCR) using the primer pair of the present invention.

In other words, it was found that *Pneumocystis jirovecii* can be detected even under the condition that 10 copies of *Pneumocystis jirovecii* as the initial amount of DNA (in the case of e) were present.

### Comparative example 1. Test of detection sensitivity in detection of Pneumocystis jirovecii using known primer pair

The detection sensitivity of *Pneumocystis jirovecii* in the nucleic acid amplification reaction (PCR) using a known primer pair was tested.

### (1) Determination of target sequence

Similar to Example 1, the base sequence (142 base pairs) represented by SEQ ID NO: 5 among the base sequences of *Pneumocystis jirovecii* was used as the target sequence.

### (2) Design and synthesis of primer

A primer pair described in Non-Patent Literature 1, that is, a forward primer pair consisting of the base sequence represented by SEQ ID NO: 3 and a reverse primer consisting of the base sequence represented by SEQ ID NO: 4 were designed and synthesized by the same method as in Example 1 (except that none of the primers used in Comparative Example 1 were labeled with the fluorescent substance).

### (3) Preparation of sample

With artificially synthesized DNA having the same base sequence as the target sequence, the DNA was diluted to 10 copies/µL, 10² copies/µL, 10³ copies/µL, 10⁴ copies/µL, and 10⁵ copies/µL in the same manner as in Example 2, thereby preparing each of the samples.

### (4) Nucleic acid amplification reaction (PCR)

Reaction solutions for PCR, containing the reagents listed in Table 3 below were prepared, and 25 µL of the samples prepared in the preparation (3) of Example 2 were added, thereby obtaining samples for PCR.

**Table 3**

| **Reagent name** | **Concentration** |
|---|---|
| Forward primer (SEQ ID NO: 3) | 0.4 µM |
| Reverse primer (SEQ ID NO: 4) | 0.4 µM |
| KOD Buffer (manufactured by Toyobo Co., Ltd.) | 1× |
| dNTP Mixture (manufactured by Toyobo Co., Ltd.) | 0.3 mM |
| KOD Hot Start (manufactured by Toyobo Co., Ltd.) | 0.06 U/µL |
| CHAPS (manufactured by Dojin Chemical Laboratory) | 0.50% |
| PEG6000 (manufactured by FUJIFILM Wako Pure Chemical Corporation) | 0.50% |
| BSA (manufactured by Sigma-Aldrich Co., LLC) | 0.1 mg/mL |
| PROCLIN 300 (manufactured by Sigma-Aldrich Co., LLC) | 0.20% |
| SYBR Green I (manufactured by LONZA Bioscience) | 1× |

Next, each of the samples for PCR was dispensed into a 96-well plate, and PCR was performed using StepOnePlus^{™} (manufactured by Applied Biosystems). After the sample was heated at 97°C for 2 minutes, the reaction was carried out such that a cycle of heating at (1) 97°C for 10 seconds → (2) 63°C for 10 seconds → (3) 72°C for 10 seconds was set as one cycle and 40 cycles of heating was performed, and the amount of fluorescence derived from the amplification product labeled in each cycle was measured.

### (5) Results

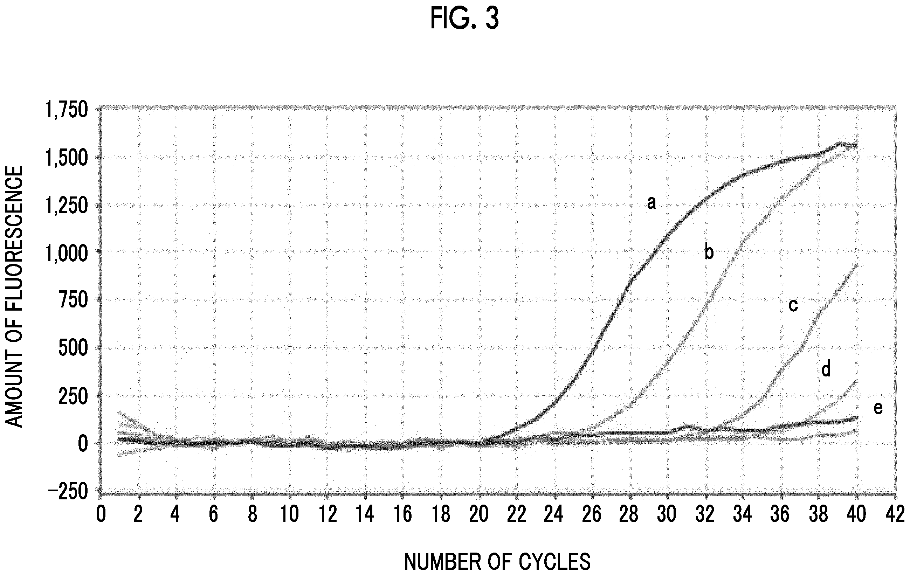

The obtained results are shown in Fig. 3. It should be noted that a to e in Fig. 3 each denote an amplification curve in a case of using samples diluted to 10⁵ copies/µL, 10⁴ copies/µL, 10³ copies/µL, 10² copies/µL, and 10 copies/µL. In addition, the vertical axis in Fig. 3 denotes the fluorescence intensity, and the horizontal axis in Fig. 3 denotes the number of PCR cycles.

As is apparent in Fig. 3, a fluorescent signal indicating the presence of the amplification product even though the fluorescence intensity was less than the fluorescence intensity of Example 2 was able to be confirmed in cases of a to d according to the nucleic acid amplification reaction (PCR) using a known primer pair. However, in the case of e (sample: 10 copies/µL), the fluorescent signal indicating the presence of the amplification product was not able to be confirmed.

In other words, it was found that *Pneumocystis jirovecii* cannot be detected under the condition that 10 copies of *Pneumocystis jirovecii* as the initial amount of DNA (in the case of e) were present using the method for detecting *Pneumocystis jirovecii* with a known primer pair.

As shown in the results of Example 2 and Comparative Example 1, it was found that *Pneumocystis jirovecii* can be detected with high sensitivity and high accuracy using the method for detecting *Pneumocystis jirovecii* with the primer pair of the present invention as compared with the detection method using a known primer pair.

### Example 3. Example 4. Test-2 of detection sensitivity in detection of Pneumocystis jirovecii using primer pair of present invention

The detection sensitivity of *Pneumocystis jirovecii* in the nucleic acid amplification reaction (PCR) using the primer pair of the present invention was tested.

### (1) Determination of target sequence

Similar to Example 1, the base sequence (142 base pairs) represented by SEQ ID NO: 5 among the base sequences of *Pneumocystis jirovecii* was used as the target sequence.

### (2) Design and synthesis of primer

A forward primer consisting of the base sequence represented by SEQ ID NO: 1 and a reverse primer consisting of the base sequence represented by SEQ ID NO: 2 were designed and synthesized in the same manner as in Example 1.

In addition, a forward primer consisting of the base sequence represented by SEQ ID NO: 3 and a reverse primer consisting of the base sequence represented by SEQ ID NO: 4 were designed and synthesized in the same manner as in Comparative Example 1 (except that none of the primers used in Example 3 and Example 4 were labeled with the fluorescent substance).

### (3) Preparation of sample

With artificially synthesized DNA having the same base sequence as the target sequence, the DNA was diluted to 10 copies/µL, 10² copies/µL, 10³ copies/µL, 10⁴ copies/µL, and 10⁵ copies/µL, thereby preparing each of the samples.

### (4) Nucleic acid amplification reaction (PCR)

Reaction solutions for PCR were prepared using the same reagents and the same method as in Example 2 except that a forward primer consisting of the base sequence represented by SEQ ID NO: 1 and a reverse primer consisting of the base sequence represented by SEQ ID NO: 4 were used as Example 3. Next, 25 µL of the samples prepared in the preparation (3) were added to the reaction solutions, thereby obtaining samples for PCR.

In addition, reaction solutions for PCR were prepared using the same reagents and the same method as in Example 2 except that a forward primer consisting of the base sequence represented by SEQ ID NO: 3 and a reverse primer consisting of the base sequence represented by SEQ ID NO: 2 were used as Example 4. Next, 25 µL of the samples prepared in the preparation (3) were added to the reaction solutions, thereby obtaining samples for PCR.

Next, each of the samples for PCR was dispensed into a 96-well plate, and PCR was performed using StepOnePlus^{™} (manufactured by Applied Biosystems). After the sample was heated at 97°C for 2 minutes, the reaction was carried out such that a cycle of heating at (1) 97°C for 10 seconds → (2) 63°C for 10 seconds → (3) 72°C for 10 seconds was set as one cycle and 40 cycles of heating was performed, and the amount of fluorescence derived from the amplification product labeled in each cycle was measured.

### (5) Results

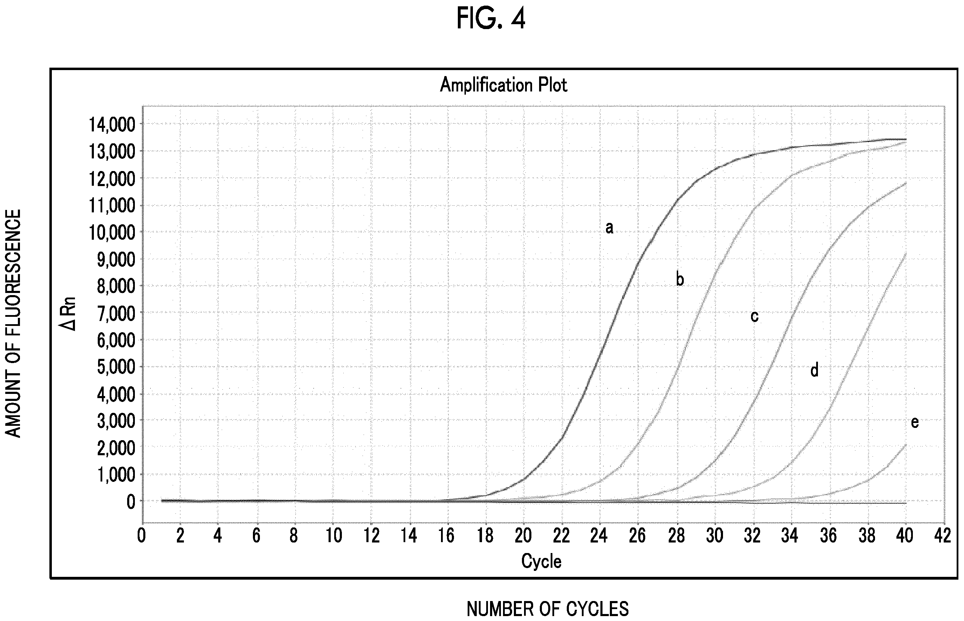

Fig. 4 shows the results obtained by performing PCR using a forward primer consisting of the base sequence represented by SEQ ID NO: 1 and a reverse primer consisting of the base sequence represented by SEQ ID NO: 4 (Example 3).

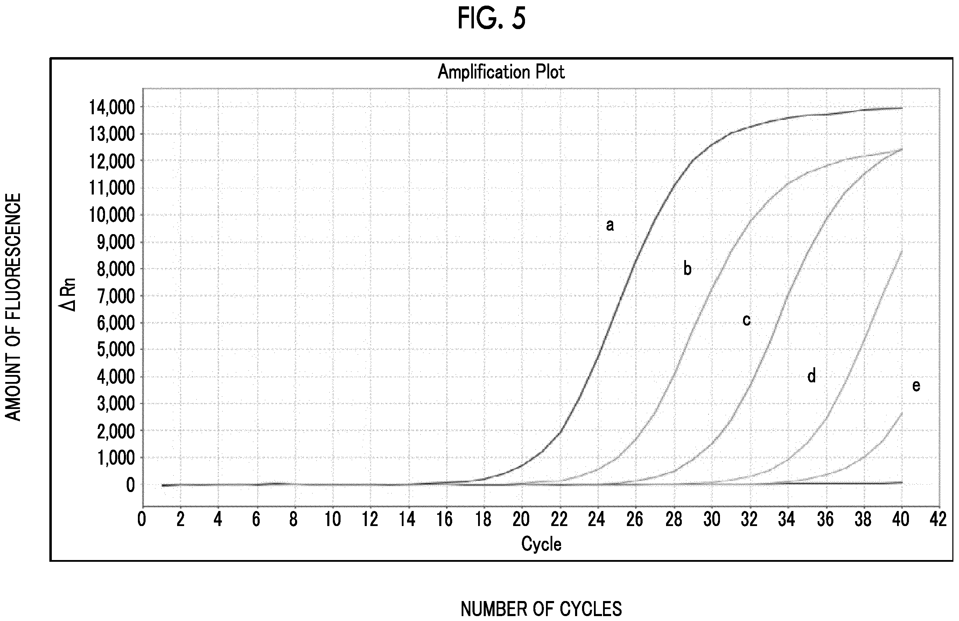

Fig. 5 shows the results obtained by performing PCR using a forward primer consisting of the base sequence represented by SEQ ID NO: 3 and a reverse primer consisting of the base sequence represented by SEQ ID NO: 2 (Example 4).

It should be noted that a to e in Figs. 4 and 5 each denote an amplification curve in a case of using samples diluted to 10⁵ copies/µL, 10⁴ copies/µL, 10³ copies/µL, 10² copies/µL, and 10 copies/µL. In addition, the vertical axis in Figs. 4 and 5 denotes the fluorescence intensity, and the horizontal axis in Figs. 4 and 5 denotes the number of PCR cycles.

As is apparent in Fig. 4, a fluorescent signal indicating the presence of the amplification product was able to be confirmed in all cases of a to e as the result of performing the nucleic acid amplification reaction (PCR) using a forward primer consisting of the base sequence represented by SEQ ID NO: 1 and a reverse primer consisting of the base sequence represented by SEQ ID NO: 4, which was the primer pair of the present invention (Example 3). In addition, as is apparent in Fig. 5, a fluorescent signal indicating the presence of the amplification product was also able to be confirmed in all cases of a to e as the result of performing the nucleic acid amplification reaction (PCR) using a forward primer consisting of the base sequence represented by SEQ ID NO: 3 and a reverse primer consisting of the base sequence represented by SEQ ID NO: 2, which was the primer pair of the present invention (Example 4). In other words, it was found that *Pneumocystis jirovecii* can be detected even under the condition that 10 copies of *Pneumocystis jirovecii* as the initial amount of DNA (in the case of e) were present in Example 3 and Example 4.

Based on the comparison of the results of Example 3 and Example 4 with the results of Comparative Example 1, it was found that *Pneumocystis jirovecii* can be detected with higher sensitivity and higher accuracy using the method for detecting *Pneumocystis jirovecii* with the primer pair of the present invention according to Example 3 and Example 4 as compared with the detection method using a known primer pair.

### Industrial Applicability

According to the method for detecting *Pneumocystis jirovecii* using the primer pair of the present invention, *Pneumocystis jirovecii* can be detected with high sensitivity and high accuracy.

## Claims

1. A primer pair for detecting *Pneumocystis jirovecii,* which is selected from the group consisting of (i) a combination of a forward primer consisting of a base sequence represented by SEQ ID NO: 1 and a reverse primer consisting of a base sequence represented by SEQ ID NO: 2, (ii) a combination of the forward primer consisting of the base sequence represented by SEQ ID NO: 1 and a reverse primer consisting of a base sequence represented by SEQ ID NO: 4, and (iii) a combination of a forward primer consisting of a base sequence represented by SEQ ID NO: 3 and the reverse primer consisting of the base sequence represented by SEQ ID NO: 2.

2. The primer pair according to claim 1,
wherein the primer pair consists of the combination of the forward primer consisting of the base sequence represented by SEQ ID NO: 1 and the reverse primer consisting of the base sequence represented by SEQ ID NO: 2.

3. The primer pair according to claim 1,
wherein at least one of the forward primer or the reverse primer is labeled with a labeling substance.

4. The primer pair according to claim 3,
wherein the labeling substance is selected from a fluorescent substance, a radioactive isotope, or an enzyme.

5. A method for detecting *Pneumocystis jirovecii,* comprising:
carrying out a nucleic acid amplification reaction using the primer pair according to claim 1 and a nucleic acid in a sample as a template, and
detecting an obtained nucleic acid amplification product.

6. A reagent kit for detecting *Pneumocystis jirovecii,* comprising the primer pair according to claim 1.
